# EUROPEAN PATENT SPECIFICATION

(11) **EP 0 423 318 B1**
(45) Date of publication and mention of the grant of the patent: **06.12.1995**
(21) Application number: 90907769.5
(22) Date of filing: 24.04.1990
(51) Int. Cl.: A61K 31/22, A61M 1/00

(54) **USE OF C5-C6 ESTERS FOR THE MANUFACTURE OF A THERAPEUTIC COMPOSITION FOR CHOLESTEROL GALLSTONE DISSOLUTION**
VERWENDUNG VON C5-C6 ESTERN ZUR HERSTELLUNG THERAPEUTISCHEN ZUSAMMENSETZUNGEN ZUR CHOLESTEROL-GALLENSTEINAUFLÖSUNG
UTILISATION DE C5-C6 ESTERS POUR L'OBTENTION D'UNE COMPOSITION THERAPEUTIQUE DESTINE A LA DISSOLUTION DE CALCULS BILIAIRES EN CHOLESTEROL

(30) Priority: 24.04.1989 US 341900
(43) Date of publication of application: 24.04.1991
(73) Proprietor: DEVELOPMENT COLLABORATIVE CORPORATION, Hamden, Connecticut 06518 (US)
(72) Inventor: HOFMANN, Alan, F., La Jolla, CA 92037 (US); SCHTEINGART, Claudio, D., La Jolla, CA 92037 (US)
(74) Representative: Wilson, Nicholas Martin
(86) International application number: US9002212
(87) International publication number: WO9012570

(56) References cited:
- EP-A- 0 251 512
- US-A- 4 758 596
- US-A- 4 902 276
- GASTROENTEROLOGY CLINICS OF NORTH AMERICA, vol. 20, no. 1, March 1991,'Pathogenesis and Therapy of Gallstone Disease, editor A.D. Cooper, pages 183-200, W.B. Saunders Co, Philadelphia, US; A.F. HOFMANN et al.: "Contact dissolution of cholesterol gallstones with organic solvents"
- JOURNAL OF PHARAMACEUTICAL SCIENCES, vol. 68, no. 9, September 1979, pages 1090-1097, American Pharmaceutical Association; G.L. FLYNN et al.: "Cholesterol solubility in organic solvents"
- THE MERCK INDEX - AN ENCYCLOPEDIA OF CHEMICALS, DRUGS, AND BIOLOGICALS, 10thedition, editor M. Windholz et al., 1983, page 749, Merck & Co.,Inc., Rahway, N.J., US;

## Description

### BACKGROUND OF THE INVENTION

### 1. Field of the Invention:

The invention herein relates to medical treatments of cholesterol calculi (particularly gallstones) by contact dissolution with organic solvents.

### 2. Background of the Prior Art:

The contact dissolution of cholesterol gallstones by organic solvents in human patients is a well recognised medical procedure and may be favoured over surgical procedures to remove gallstones in patients at increased risk of surgery; see, for instance, US Patent No. 4 205 086. The dissolution procedures normally involve infusion of the solvent into the biliary tract (including the gallbladder and the bile ducts) by means of a T-tube, nasobiliary tube, percutaneous transhepatic catheter or cholecystostomy tube by use of a constant infusion pump or by gravity or by manual repeated installation and withdrawal using a syringe; see Palmer, et al., Gut 27, 2, 196 (1986). Often the stones fragment during the dissolution procedure, which advantageously increases the rate of dissolution.

A number of different types of solvents have been used or suggested for the dissolution procedure. These include organic solvents such as diethyl ether, chloroform or d-limonene as well as aqueous micellar solutions of bile salts. The aforementioned US Patent No. 4 205 086 also lists a large number of useful liquid fatty acids and the alcohol esters thereof. A further solvent which has received substantial attention is monooctanoin, mentioned in US Patent No. 4 755 167 and several articles, such as Thistle, et al., Gastroenterology, 78 5, 1016 (1980). More recently methyl t-butyl ether (MTBE) has been used as a cholesterol gallstone solvent; see eg. Allen, et al., Gastroenterology, 88, 1, 122 (1895); Thistle, et al., US Patent No. 4 758 596 and Thistle, et al., N. Engl. J. Med. 320, 633 (1989).

While all of these materials have shown some efficacy in in vivo and/or in vitro tests, all have some undesirable side effects on physical properties. Monooctanoin, for instance, has a relatively high viscosity and dissolves gallstones very slowly. MTBE was selected by Thistle, et al., because its boiling point is above body temperature. However, if MTBE escapes from the gallbladder into the small intestine, it causes signs of systemic toxicity (sedation), pain, nausea, and by endoscopy, damage to the epithelium of the small intestine. Entry of MTBE into the blood stream causes hemolysis, and one case of renal failure (reversible) has been reported. MTBE also has a relatively low boiling point (55°C), not far above ordinary body temperature, which poses some volatility problems in use because of its low flash point and strong, unpleasant odour. Those materials such as diethyl ether which have boiling points below body temperature are hazardous for clinical use because of rapid volatilization and marked increase in volume in use.

US 4 758 596 discloses a method of dissolving cholesterol calculus in vitro or in vivo by contacting the cholesterol calculus with methyl tertiary-butyl ether. EP-A1-0251512 disclosed a pressure sensitive apparatus for removing obstructions in bodily cavities or organs by delivering and receiving fluid to effect dissolution and removal of the obstruction of, for example gallstones. In Gastroenterology Clinics of North America, vol. 20, No. 1, 1991 "Pathogenesis and therapy of gallstone disease, pages 183-200, W.B. Saunders & Co, Philadelphia, A.F. Hofmann discusses the contact dissolution of symptomatic cholesterol gallstones with organic solvents. The solubility of cholesterol in organic solvents is discussed in the article by G.L. Flynn in J. Am. Pharm.Sci., Vol. 68, No. 9, September 1979.

Recently there has been developed a novel infusion pump which produces a high flow rate at low pressure. This pump has been described and claimed in US Patent No. 4 902 276 issued 20 February 1990. While this pump has proved very effective for gallstone dissolution, its satisfactory performance depends on being used with relatively low viscosity solvents.

It would therefore be advantageous to have a therapeutic procedure available in which cholesterol gallstones could be easily, safely and rapidly dissolved by a solvent which would be effective for dissolution or cholesterol gallstones, easy and safe to handle for medical personnel and capable of being used in the most effective equipment such as the Zakko pump.

### BRIEF SUMMARY OF THE INVENTION

The invention provides for the use of a solvent comprising a C₅-C₆ ester having a boiling point in the range of 80-140°C for manufacture of a pharmaceutical composition for dissolving cholesterol calculi in the biliary duct of a patient.

Particularly preferred among the C₅-C₆ esters are those selected from the group consisting of n-propyl acetate, isopropyl acetate, ethyl propionate, ethyl isobutyrate. These have all been found to produce rapid dissolution of cholesterol gallstones (at a rate generally comparable to that of MTBE) without problems of volatility or excessive viscosity.

The solvent may be in an undiluted form or diluted by an inert carrier liquid.

### DETAILED DESCRIPTION AND PREFERRED EMBODIMENTS

The efficiency of the use of the solvent of this invention can be best understood by consideration of the characteristics of cholesterol calculi and the claimed class of solvents useful in the invention.

As used herein, the term "cholesterol calculi" means those concretions which generally develop in hollow organs or ducts within humans and animals and which contain at least about 40% cholesterol. Well known common cholesterol calculi are cholesterol biliary duct stones and gallstones. Also, the term "biliary tract" will be recognised to encompass both the gallbladder and the bile ducts.

Consideration of the usual equations used to describe dissolution of a sphere by a fluid under turbulent conditions indicates that not only equilibrium solubility but also viscosity is important. This is because viscosity influences the diffusion of cholesterol in the unstirred layer immediately surrounding the stone (according to the Stokes-Einstein relationship). In addition, low viscosity is important since catheters used for this purpose must have a small bore (usually less than 8 French 2.64 mm diameter). with a high flow-low pressure pumping system such as that of the Zakko Pump. The pressure needed to obtain a given flow varies inversely as the 4th power of the radius, and directly with the viscosity of the fluid being pumped. Thus for a small bore tube, the lower the viscosity, the greater is the solvent flow for a given pressure.

There are two additional requirements for a satisfactory solvent. It must be relatively non-toxic in several aspects. First, the solvent must not damage the gallbladder mucosa significantly during a several hour exposure of the mucosa to the anhydrous solvent. Second, the solvent must not damage the mucosa of the biliary duct system or the small intestine if the solvent leaks out of the gallbladder. The solvent should cause little hemolysis if mixed with blood, should not cause release of toxic cytokines such as platelet activating factor, and should not be toxic if it escapes into the peritoneal cavity. In addition, the solvent should have a pleasant odor so that if absorbed it does not induce nausea. Further, the solvent should be rapidly metabolized by digestive enzymes if it escapes into the gastrointestinal tract, and the resultant digestive products should be known to be safe.

Lastly, there are safety considerations in handling the solvent. If spilled, it should not cause danger to exposed employees. It should be a non-explosive and have a relatively high flash point. It should have a stable shelf life and not form peroxides or other explosive by-products.

Viscosity is known to vary directly with the number of carbon atoms in a molecule. It varies inversely approximately exponentially with temperature. Boiling point and flash point also vary directly with the total number of carbon atoms, but the relationship is not as simple. Cholesterol solubility in solvents can be predicted to some extent by solubility parameter considerations.

The determination of solvents suitable for effective contact dissolution of cholesterol gallstones thus involves multiple considerations. In addition, there are solute considerations. Cholesterol gallstones are composed of cholesterol monohydrate (and other insoluble salts); see Whiting, et al., Clin. Sci., 68, 589 (1985). If gallstones are stored in the dry state, the water may evaporate so that the cholesterol monohydrate becomes anhydrous cholesterol. Therefore testing of solvents is best done on freshly harvested human gallstones which have been kept wet.

Based on these considerations, our research has led to the discovery that effective solvents are those having 5-6 carbon atoms per molecule and being selected from the group consisting of ethers, esters and ketones with a boiling point in the range of 80°-140°C and mixtures thereof. We have also discovered that within this class those compounds which are particularly preferred solvents, due to their most closely meeting the desirable criteria discussed above, are n-propyl acetate, isopropyl acetate, ethyl propionate, ethyl isobutyrate, cyclopentanone, 3-pentanone and methyl t-amyl ether (methyl 1,1-dimethylpropyl ether). All of these are known compounds and need not be defined further here; their physical and chemical properties are well described in the chemical literature.

Their efficacy may be seen from the following data. In the tests described in Table I below, fresh human faceted cholesterol gallstones (kept in distilled water) were dissolved in vitro and the times were recorded for the total dissolution, using a model gallbladder at a high flow rate achieved by a metering pump. "High flow rate" as used herein will mean rates of flow in the range which can be anticipated to result in fluid turbulence for the solvent in the region of the biliary tract surrounding the calculi (gallstones). While "high" rates will vary according to solvent type and viscosity and with individual patients, it will be expected that appropriate flow rates will be at least about 20 ml/min, preferably at least about 100 ml/min, and more preferably at least about 150 ml/min. For comparison purposes a parallel test with MTBE is also shown. The second column shows the weight of stone treated, of which 90% by weight can be considered to be cholesterol.

**TABLE I**

| Solvent | Boiling Point, °C | Stone Weight, mg | Dissolution | |
|---|---|---|---|---|
| | | | Time, min. | Rate, mg/min. |
| n-propyl acetate | 102 | 150 | 10 | 14 |
| isopropyl acetate | 89 | 147 | 14 | 9 |
| ethyl propionate | 99 | 140 | 10 | 13 |
| ethyl isobutyrate | 112 | 132 | 15 | 8 |
| cyclopentanone | 131 | 125 | 10 | 11 |
| 3-pentanone | 101 | 142 | 9 | 14 |
| methyl t-amyl ether | 85 | 131 | 8 | 15 |
| methyl t-butyl ether | 55 | 175 | 8 | 20 |

It will be evident from these data that the C₅-C₆ ether, ester and ketone solvents of this invention are essentially similar to the known MTBE in their ability to rapidly dissolve cholesterol gallstones, while yet having substantially higher boiling points than MTBE and thus substantially less potential for problems with volatility.

Toxicological considerations indicate that the first six solvents have a low order of toxicity, although only limited information is available on cyclopentanone. The toxicity of methyl t-amyl ether has not been studied, but should be similar to that of MTBE; see Savolainen, et al., Arch. Toxicol., 57, 285 (1985).

In another series of experiments, the effectiveness of propyl acetate, isopropyl acetate, ethyl propionate and ethyl isobutyrate esters versus MTBE in dissolving cholesterol gallstones was determined in vitro. For this study stones from five cholecystectomy patients were matched. All stones were visually judged to be primarily cholesterol. The mean stone weight for the test sets (five stones each) ranged from 79-340 mg. The test protocol utilized a 30 ml polyethylene bag as a model gallbladder. One stone was dissolved at a time. For each stone a reservoir containing 150 ml of solvent was used. Flow to the bag was maintained at 180 ml/min. A closed loop pumping system was employed to deliver the solvent to the experimental stone within the polyethylene bag. A Medical Disposables International subclavian catheter (#1900115A) was used to deliver and withdraw the solvent and monitor pressure which controlled the pump. This catheter consisted of two 18 gage lumens (6 mm diameter) and a single 16 gage lumens (5 1/3 mm diameter). The larger lumens were used to aspirate and infuse the solvent and the smaller lumen was used to monitor pressure within the model gallbladder. The termination of each lumen was a single port. The feed back from the pressure transducer was used to control the pumps to delivery 180 ml/min within a predetermined intralumen pressure. A flow of 180 ml/min was used to assure high turbulence and obtain optimum dissolution rates due to high rates of mass transfer. Complete dissolution was defined as the disappearance of all debris in the model gallbladder. The data are summarized in Table II.

**TABLE II**

| | Mean Time min. | Overall Rate mg/min. |
|---|---|---|
| Ethyl Propionate | 14 | 12.22 |
| MTBE | 19 | 7.69 |
| Isopropyl Acetate | 26 | 5.48 |
| Ethyl Isobutyrate | 33 | 4.55 |
| Propyl Acetate | 35 | 4.45 |

This comparison shows the esters tested to be substantially equivalent to MTBE. The variation in rates is well within experimental expectations, since great differences exist from stone to stone for each solvent and the surface area per unit volume increases as the stone diameter (weight) decreases for similar materials and for similar geometries. It is also expected that the chemical differences from stone to stone may cause one solvent to perform appreciably different than the next.

Metabolic considerations indicate that the esters (n-propyl acetate, isopropyl acetate, ethyl propionate and ethyl isobutyrate) are likely to be hydrolyzed by digestive esterases and quickly converted to the corresponding alkanol and aliphatic acid (which will ionize). The hydrolysis products -- ethanol, n-propanol and isopropanol and the acetate, propionate and butyrate ions -- all have a low degree of toxicity. The ketones will be reduced in the liver to the equivalent alcohols, which are generally non-toxic.

We believe that it is likely that a mixture the solvents will have less toxicity (expressed per unit volume of solvent) than any of the solvents alone because each of the solvents undergoes a different metabolic pathway.

Any suitable technique of infusion of the described solvents into a patient can be used; normally the one chosen will be as that described in the prior art for other solvents,, including monooctanoin and MTBE. The solvents are normally used in undiluted liquid form, but if desired may also be used as part of mixtures with inert liquid carriers. Since dissolution is a contact phenomenon it will be recognized that dilution in such mixtures will normally slow the rate of dissolution and extend the time needed to achieve the desired degree of gallstone dissolution.

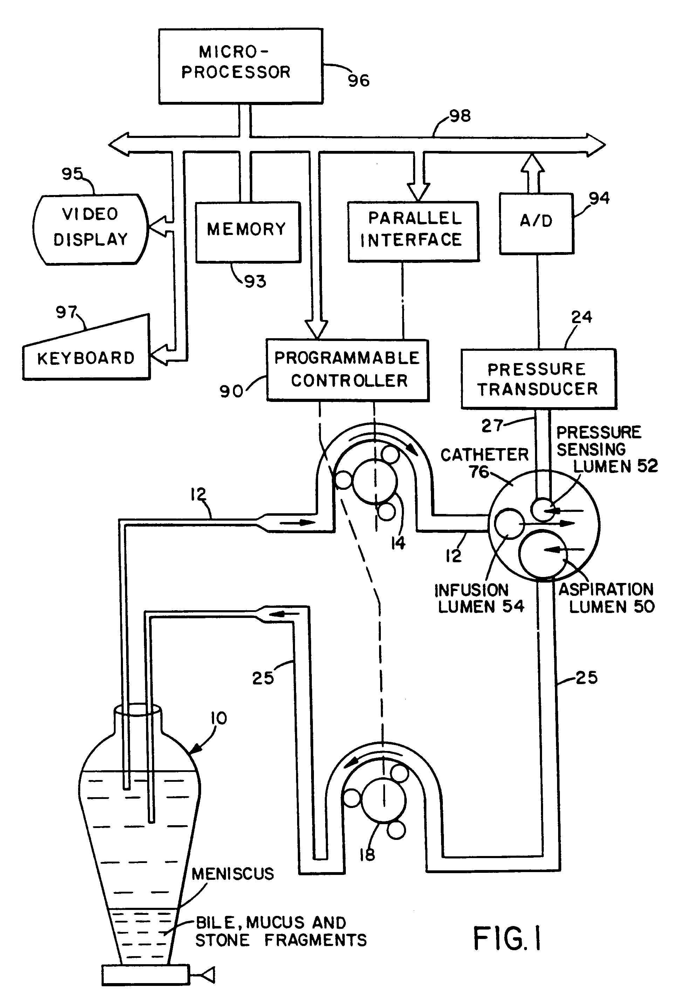

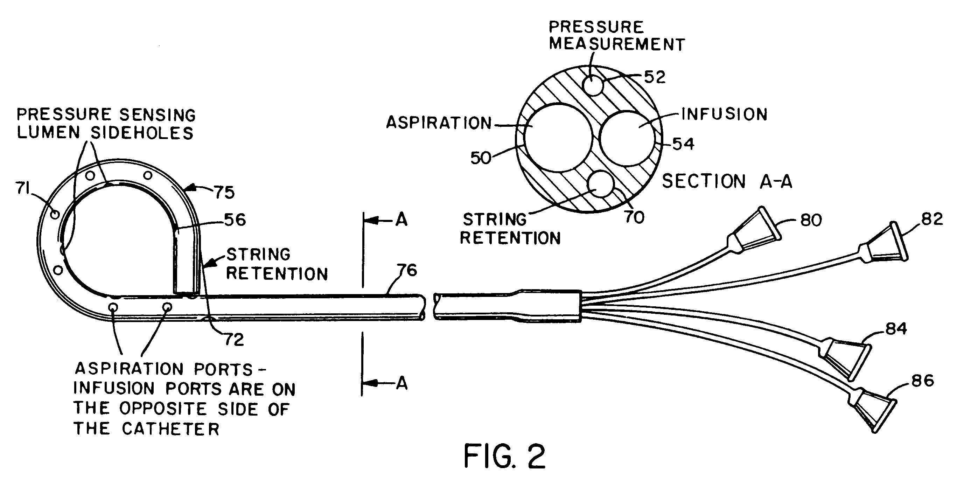

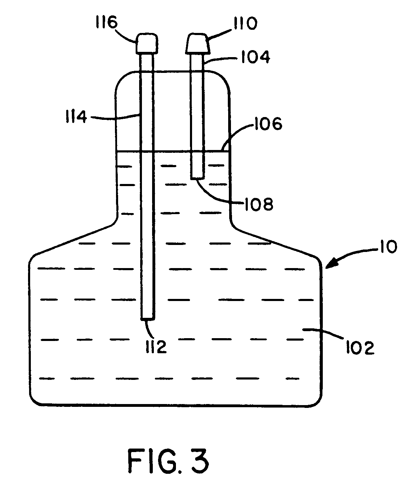

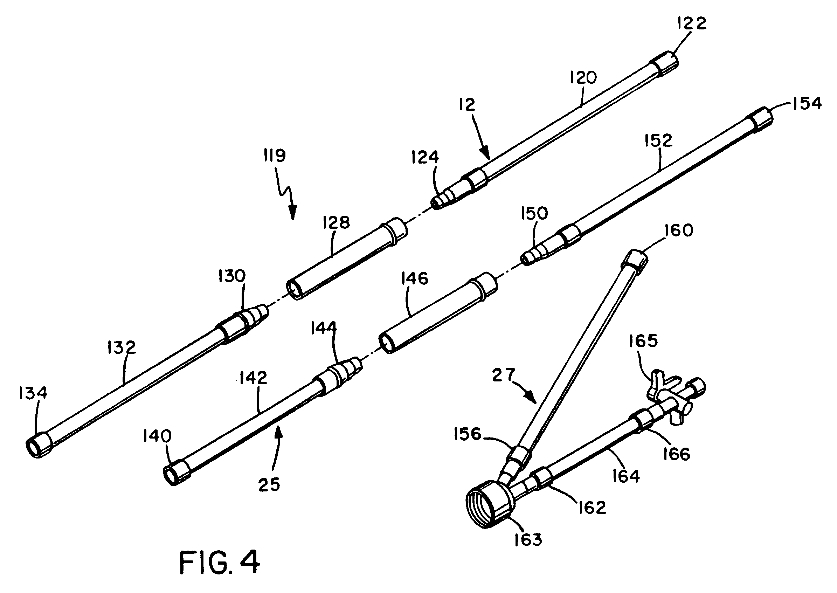

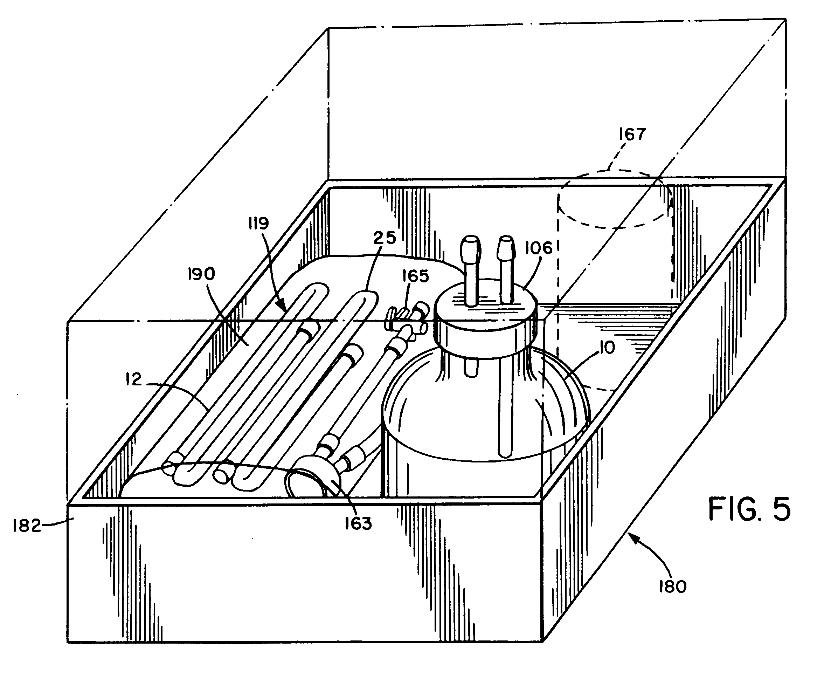

## Claims

1. The use of a solvent comprising a C₅-C₆ ester having a boiling point in the range of 80°-140°C for manufacture of a pharmaceutical composition for dissolving cholesterol calculi in the biliary duct of a patient.

2. The use of a solvent as in Claim 1, wherein said ester is n-propyl acetate, isopropyl acetate, ethyl propionate or ethyl isobutyrate.

3. The use of a solvent as claimed in Claim 1, wherein said solvent is used in an undiluted form.

4. The use of a solvent as claimed in Claim 1, wherein said solvent is used in a form in which it is mixed with and diluted by an inert carrier liquid.

## Patentansprüche

1. Verwendung eines Lösungsmittels, das einen C₅-C₆ Ester mit einem Siedepunkt im Bereich von 80° bis 140°C enthält, zur Herstellung einer pharmazeutischen Zusammensetzung zum Auflösen von Cholesterinsteinen im Gallengang eines Patienten.

2. Verwendung eines Lösungsmittels nach Anspruch 1, wobei der genannte Ester n-Propylacetat, Isopropylacetat, Ethylpropionat oder Ethylisobutyrat ist.

3. Verwendung eines Lösungsmittels nach Anspruch 1, wobei das genannte Lösungsmittel in unverdünnter Form verwendet wird.

4. Verwendung eines Lösungsmittels nach Anspruch 1, wobei das Lösungsmittel in einer Form verwendet wird, in welcher es mit einer inerten Trägerflüssigkeit gemischt und durch diese verdünnt wird.

## Revendications

1. Utilisation d'un solvant comprenant un ester en C₅-C₆ ayant un point d'ébullition dans la gamme de 80°-140°C pour la fabrication d'une composition pharmaceutique destinée à la dissolution de calculs de cholestérol dans la voie biliaire d'un malade.

2. Utilisation d'un solvant selon la revendication 1, caractérisée en ce que ledit ester est l'acétate de n-propyle, l'acétate d'isopropyle, le propionate d'éthyle ou l'isobutyrate d'éthyle.

3. Utilisation d'un solvant selon la revendication 1, caractérisée en ce que ledit solvant est utilisé sous forme non diluée.

4. Utilisation d'un solvant selon la revendication 1, caractérisée en ce que ledit solvant est utilisé sous une forme dans laquelle il est mélangé avec et dilué par un support liquide inerte.
